# EUROPÄISCHE PATENTSCHRIFT

(11) **EP 0 758 636 B1**
(45) Veröffentlichungstag und Bekanntmachung des Hinweises auf die Patenterteilung: **05.01.2000**
(21) Anmeldenummer: 96112423.7
(22) Anmeldetag: 01.08.1996
(51) Int. Cl.: C07C 37/72, C07C 37/82, C02F 1/26, C02F 1/28, B01D 11/04, B01D 15/00

(54) **Verfahren zur Reinigung des Prozessabwassers des Bisphenol-A-Verfahrens**
Process for the treatment of waste water from a bisphenol-A preparation process
Procédé pour le traitement des eaux usées du procédé de préparation du bisphénol-A

(30) Priorität: 14.08.1995 DE 19529854; 06.09.1995 DE 19532888
(43) Veröffentlichungstag der Anmeldung: 19.02.1997
(73) Patentinhaber: BAYER AG, 51368 Leverkusen (DE)
(72) Erfinder: Meurer, Kurt-Peter, Dr., 51375 Leverkusen (DE); Van Osselaer, Tony, Dr., 9100 Belsele (BE); Hinz, Jürgen, Dr., 47800 Krefeld (DE); Augustin, Thomas, Dr., 50735 Köln (DE)

(56) Entgegenhaltungen:
- US-A- 4 374 283
- US-A- 4 400 553
- US-A- 4 469 561

## Beschreibung

Die Erfindungs betrifft ein Verfahren zur kontinuierlichen in-process-Reinigung von organisch belasteten Prozeß-Abwässern der Herstellung von Bisphenol-A.

Die 5 Gew % bis 10 Gew % Phenol oder andere Edukte oder Produkte des BPA-Verfahrens enthaltenden Prozeß-Abwässer werden mit Hilfe eines kontinuierlichen Lösungsmittel-Extraktionsverfahrens mit nachgeschalteter Adsorptionsanlage so gereinigt, daß die TOC-Belastung kleiner als 50 ppm und die Phenol-Belastung kleiner als 3 ppm sind.

Im Bisphenol-A-Verfahren (Ionenaustauscherverfahren, s. DE-A 3 727 641, US-A 4 912 263, DE-A 4 105 428, DE-A 4 213 872) fallen etwa 7 bis 8 % Phenol und andere Edukte oder Produkte des BPA-Verfahrens enthaltende Prozeßabwässer an, die einer Abwasseraufarbeitung zugeführt werden müssen.

US-A 4,469,561 offenbart ein Verfahren zur Abwasseraufbereitung, bei dem die Prozeß-abwässer aus der Bisphenolherstellung mit Toluol extrahiert werden; in US-A 4,374,283 und US-A 4,400,553 wird Methylisobutylketon (MIBK) als Extraktionsmittel eingesetzt. Weiterhin lehren diese Schriften, daß die so aufbereiteten Abwässer durch Behandlung mit Aktivkohle oder einem (nicht näher spezifizierten) organischen Harz weiter gereinigt werden können.

Im Bisphenol-Verfahren wird in einer Phenolwasserextraktion das organisch hochbelastete Prozeßwasser auf TOC-Werte kleiner als 500 ppm und Phenolwerte kleiner als 50 ppm gereinigt. Es treten jedoch Betriebssituationen auf, bei denen höhere Werte vorkommen können.

Die Erfindung betrifft ein Verfahren, bei dem nach der Vorreinigung des Prozeßwassers durch die betriebseigene Phenolwasserextraktions-Anlage das vorgereinigte Prozeßwasser über eine nachgeschaltete Adsorptionsanlage weiter gereinigt wird.

Die TOC-Werte sind dann kleiner als 50 ppm und der Phenolgehalt ist kaum noch analytisch bestimmbar (< 1 ppm).

Das auf diese Weise hochgereinigte Wasser muß dann nicht mehr der werkseigenen Abwasseraufbereitung zugeführt werden.

Dieses organisch nicht mehr belastete Wasser kann als Rohstoff wieder in anderen Prozessen verwendet werden oder direkt in die Umwelt abgegeben werden.

Gegenstand der Erfindung ist ein kontinuierliches Verfahren zur Aufbereitung von Phenol und andere Edukte oder Produkte des BPA-Verfahrens enthaltenden Prozeßabwässern des Bisphenol-A-Verfahrens, dadurch gekennzeichnet, daß eine in-process-Reinigung der Prozeßabwässer in zwei Reinigungsschritten in der Weise erfolgt, daß zunächst Prozeßabwässer mit einem Gehalt an Phenol und anderer Edukte und Produkte des BPA-Verfahrens von 2 Gew % bis 20 Gew %, bevorzugt 5 Gew %, in ein kontinuierlich betriebenes Lösungsmittelextraktionsverfahren eingespeist werden, die Reinigung durch eine im Gegenstrom betriebene Extraktion der wäßrigen Phase mit einem organischen Lösungsmittel erfolgt, wobei als Extraktionsmittel bevorzugt Methylisobutylketon eingesetzt wird, daß die so gereinigten Prozeßabwässer einen Gehalt an Phenol kleiner als 250 ppm und einen TOC-Wert kleiner als 500 ppm besitzen, daß das extrahierte Phenol und die zurückgewonnen Leichtsieder wie z.B. Aceton in den Bisphenol-Prozeß zurückgeführt werden, daß das Extraktionsmittel wiederaufgearbeitet und in den Extraktionsprozeß wieder zurückgeführt wird, daß in der Extraktionsanlage vorgereinigten Prozeßabwässer in eine mit Polystyrolharz gefüllte Adsorptionsanlage geführt werden.

Die so hochgereinigten Prozeßabwasser haben einen Phenol-Gehalt kleiner als 3 ppm und eine TOC-Belastung kleiner als 50 ppm. Sie können direkt in die Umwelt abgegeben oder als Rohstoff in Entsalzungsanlagen zur Herstellung von demineralisiertem Wasser bzw. in anderen Prozessen verwendet werden. Das von organischen Verunreinigungen befreite, allerdings Salze wie Sulfate enthaltende Prozeßwasser kann z.B. als Rohstoff zur Verdünnung hochkonzentrierter Natronlauge dienen, die wiederum als Rohstoff zur Herstellung hochreiner Natriumbisphenolat-Lösungen eingesetzt werden. Die Natriumbisphenolat-Lösung ist ein wichtiges Edukt zur Herstellung hochtransparenter Polycarbonate nach dem Phasengrenzflächenverfahren.

Mit diesem kombinierten Extraktions-/Adsorptionsverfahren wird eine Aufbereitung des Prozeßwassers in einer zentralen Kläranlage vermieden. Zudem wird das Bisphenol-Verfahren hinsichtlich jährlich anfallender Abwasserkosten entlastet und unter ökologischen Gesichtspunkten deutlich verbessert.

### Beispiele

### Beispiel 1

5 to/h Prozeßabwasser mit einem Gehalt an Phenol von 4,8 Gew % Phenol und einem Gehalt an Aceton von 0,1 % und einem Gehalt an Methanol von 800 ppm wurde unter Zudosierung von verdünnter H₂SO₄ auf einen pH-Wert von pH = 1 eingestellt. Diese Lösung wurde im Gegenstrom mit einem gleichen Volumen an Methylisobutylketon extrahiert. Das Sumpfwasser wurde auf TOC und Phenol geprüft. Es wurden TOC-Werte von 150 ppm und Phenolgehalte von 30 ppm gemessen.

Phenol enthaltendes Methylisobutylketon wurde destillativ von Phenol getrennt, aufdestilliert und im Extraktionsprozeß wieder eingesetzt. Das zurückgewonnene Phenol wurde in den Prozeß zurückgeführt, aufdestilliert und im Prozeß wieder eingesetzt.

Das so gereinigte Prozeßabwasser wurde auf einen bei Raumtemperatur betriebenen, mit Polystyrolharz gefüllten Adsorber geführt. Das erhaltene hochgereinigte Prozeßabwasser wies Phenol-Werte <1 ppm und TOC-Werte um 20 ppm auf.

Die Regeneration des Adsorbers wurde nach 5 Tagen durchgeführt, der Phenoldurchschlag betrug 15 ppm.

### Beispiel 2

Beispiel 1 wurde mit einem 8 Gew.-% Phenol enthaltenden Prozeßabwasser wiederholt. Nach dem ersten Reinigungsschritt wurden TOC-Werte von 120 ppm und Phenolgehalte von 25 ppm gemessen. Nach dem zweiten Reinigungsschritt wurden TOC-Werte von 25 ppm und Phenolgehalte <2 ppm gemessen. Die Regeneration des Adsorbers wurde nach 4 Tagen durchgeführt.

## Patentansprüche

1. Kontinuierliches Verfahren zur Aufbereitung von Phenol und andere Edukte und Produkte des BPA-Verfahrens enthaltenden Prozeßabwässern des Bisphenol-A-Verfahrens, dadurch gekennzeichnet, daß eine in-process-Reinigung der Prozeß-Abwässer in zwei Reinigungsschritten in der Weise erfolgt, daß zunächst Prozeßabwässer mit einem Gehalt an Phenol und anderen Edukten und Produkten des BPA-Verfahrens von 2 Gew % bis 20 Gew % in ein kontinuierlich betriebenes Lösungsmittelextraktionsverfahren eingespeist werden, die Reinigung durch eine im Gegenstrom betriebene Extraktion der wäßrigen Phase mit einem organischen Lösungsmittel erfolgt, daß die so gereinigten Prozeßabwässer einen Gehalt an Phenol kleiner als 250 ppm und einen TOC-Wert kleiner als 500 ppm besitzen, daß das extrahierte Phenol und die zurückgewonnen Leichtsieder in den Bisphenol-Prozeß zurückgeführt werden, daß das Extraktionsmittel wiederaufgearbeitet und in den Extraktionsprozeß wieder zurückgeführt wird, und daß anschließend die in der Extraktionsanlage vorgereinigten Prozeßabwässer in eine mit Polystyrolharz gefüllte Adsorptionsanlage geführt werden.

## Claims

1. Continuous process for the treatment of waste water from the bisphenol A process containing phenol and other educts and products of the BPA process, characterised in that an in-process purification of the process waste water is carried out in two purification steps in such a way that, first of all, process waste water containing phenol and other educts and products of the BPA process in quantities of 2 wt.% to 20 wt.% is fed into a continuously operated solvent extraction process and the purification is carried out by a counter-current extraction of the aqueous phase by means of an organic solvent, that the process waste water thus purified has a phenol content of less than 250 ppm and a TOC value of less than 500 ppm, that the extracted phenol and the recovered low-boiling components are returned to the bisphenol process, that the extracting agent is retreated and returned to the extraction process, and that subsequently the process waste water previously purified in the extraction plant is passed to an adsorption unit filled with polystyrene resin.

## Revendications

1. Procédé en continu pour le traitement d'eaux usées issues du procédé de préparation du Bisphénol-A contenant du phénol, ainsi que d'autres éduits et produits du procédé de préparation du BPA, caractérisé en ce qu'on procède à une purification en cours de fabrication des eaux usées issues du procédé, dans deux étapes de purification, de telle sorte que l'on introduit d'abord des eaux usées issues du procédé dont la teneur en phénol et en autres éduits et produits issus du procédé de préparation du BPA s'élève de 2% en poids à 20% en poids dans un procédé d'extraction dans un solvant mis en oeuvre en continu, la purification étant réalisée via une extraction de la phase aqueuse dans un solvant organique, mise en oeuvre à contre-courant, que les eaux usées issues du procédé ainsi purifiées possèdent une teneur en phénol inférieure à 250 ppm et une teneur totale en carbone organique inférieure à 500 ppm, que l'on renvoie dans le procédé de préparation du bisphénol le phénol obtenu par extraction et les fractions à bas points d'ébullition récupérées, que l'on soumet l'agent d'extraction à un traitement ultérieur et on le renvoie à nouveau'dans le procédé d'extraction, et que l'on guide ultérieurement les eaux usées issues du procédé soumises à une purification préalable dans l'installation d'extraction, dans une installation d'adsorption garnie avec de la résine de polystyrène.
